# EUROPEAN PATENT APPLICATION

(11) **EP 2 896 608 A1**
(43) Date of publication of application: **22.07.2015**
(21) Application number: 15000067.7
(22) Date of filing: 14.01.2015
(51) Int. Cl.: C07C 29/76

(54) **Process for the purification of crude glycerin**

(30) Priority: 15.01.2014 BR 102014001003
(71) Applicant: Glycerosolution Quimica, Ltda, CEP 04571-000 Sao Paulo (SP) (BR)
(72) Inventor: Bombardi Ferreira, Josse Givan, 15115-000 Bady Bassitt (SP) (BR); Borges Sato, Marcelo Eiji, 05706-290 Sao Paulo (SP) (BR); Sato, Setsuo, 05706.290 São Paulo - SP (BR); Cazzolato, Silvio Antonio, 12242.431 Sao José dos Campos, SP (BR); Meira DA Silva, Taciane, 13515-000 Charqueada, SP (BR)
(74) Representative: Juncosa Miro, Jaime

(57) **Abstract**

Process of crude glycerin purification originated from transesterifications with alkaline catalysis without using acidification and distillation producing purified glycerin 96% and 99% purity. The mechanism is the interaction of liquid/liquid extraction in batch system or continuous form and the relative mass balance between the components existent in glycerin in the crude form (without mineral acidification). The technique use the molecular interactions between the components of crude glycerin generated in the process of transesterification, compounds of free glycerin, alkaline soaps of fatty acids, fatty esters, monoglycerides, diglycerides, water-free methanol, where this mixture usually is in a single homogeneous phase. This composition is unbalanced creating a light phase and a heavy phase. The separated glycerin is free of salts and the light part is treated with organic or inorganic acids to transform the fatty soaps into free fatty acids, which are used to make the unbalance of homogeneous crude glycerin.

## Description

### Field of the invention

The present invention presents a new purification technique of glycerin generated in the processes of transesterification of triglycerides that use alkaline catalysis, oxides, hydroxides, sodium alkoxides, potassium, lithium. Such processes are used in large scale for production of biodiesel and industrial esters. A technique used aims to take advantage of existing molecular interactions between components of crude glycerin generated in the process of transesterification, compounds of free glycerin, alkaline soaps of fatty acids, fatty esters, monoglycerides, diglycerides, water free methanol, where this mixture is usually in a single homogeneous phase. The very simple but very innovative developed technique consists of modifying this composition with very specific relations so that this homogeneous balance is unbalanced creating a light phase composed of soaps, esters, mono- and diglycerides, and a heavy phase composed of free glycerin, methanol and water. With this technique, the separated glycerin is free of salts and the light part is treated with organic or inorganic acids to transform the fatty soaps into free fatty acids, which are used to make the unbalance of homogeneous crude glycerin. The glycerin phase after removal of methanol and water has purity above 96%, free of salts.

### Background of the invention

The glycerin is a compound whose scientific name 1,2,3-propanetriol naturally occurs in animal oils and fats combined in the form of triglycerides. After leaving the production process, it is presented as an oily, colorless and viscous liquid and sweet taste, soluble in water and alcohol and insoluble in hydrocarbon, has melting point of 17.8°C and its evaporation with respective decomposition occurs at 290°C.

Currently its production is obtained by saponification of oils and fats, synthetically as secondary product of propylene manufacturing, and also as a byproduct of transesterification to form fatty esters (approximately 10% of mass of the crude material used), which technique used for production of biodiesel is presented as innovative form in the invention EP 13152268-2, in which the maximum production of biodiesel is aimed. This glycerin obtained has low cost and high quality.

In the present times, biodiesel emerged as a viable alternative in terms of renewable fuel. The transesterification is the currently most used process for the production of biodiesel. It consists of a chemical reaction of the vegetable oils or animal fats with common alcohol (ethanol) or methanol stimulated by a catalyst, from which the crude or blond glycerin is also extracted, which after its distillation becomes input used with several applications in the chemical industry.

As with biodiesel, the catalysts interfere in the production and quality of glycerin produced. Among the used, there are many types as homogeneous, heterogeneous, and enzymatic, as they can be de acid and basic. The basic homogenous catalysts are more used (CH₃ONa, CH₃OK, CH₃OLi, NAOH, KOH, LiOH) for allowing high reactivity and efficiency at low temperature, around 60°C, and relativity low cost. The disadvantage of this method is the formation of fatty soaps and mineral salts, forcing this glycerin to be distillation, an expensive and high-energy consumption technique, to have industrial use. Already the acid catalysis is used when the reaction mean has a high acidity index, this method has a good yield; however, there is the problem of corrosion and it is needed to be done in high temperatures using great excess of alcohol (usually anhydrous methanol or ethanol). The heterogeneous catalysts (calcium oxide, magnesium oxide, zirconium, aluminum, niobium and their derivatives, supported tin-based catalysts or not) are removed more easily from reaction mixture and can minimize the environmental problems, apart from being easily removed from reaction medium, but they work in high temperatures and/or use excess of alcohol much larger when compared with catalysis via alkaline alkoxides. Already, the enzymatic catalysts allow understanding an easy separation between the biodiesel and glycerin, the glycerin produced by this mean is of high purity. The great restriction of this technique is the excess of reagents used, cost of enzymes, mostly the ones supported that can be recycled, and a reaction conversion rate maximum of 94%, requiring the use of other methods to achieve 99% of conversion to achieve the quality indexes required by biodiesel regulation. The use of some buffers to regulate the pH of enzymatic reaction medium, many of them based on sodium salts end up contaminating crude glycerin.

The glycerin generated in the biodiesel Production (homogeneous alkaline catalysis) has purity from 60 to 80%, but the great inconvenient consists of the presence of inorganic salts as sodium sulfate and sodium chloride making the process of glycerin purification expensive and with corrosion problems in the equipment used to distill the glycerin. The use of this glycerin such as is very limited due to the presence of these salts. In addition to the productive aspect, there is the formation of large amounts of solid waste that must be disposed into environment. With all these negative aspects, the value of this glycerin is marginal, currently being a problem for the great biodiesel producers.

Several techniques of purification of this glycerin have been practiced, among them it is highlighted the distillation of glycerin in high vacuum, in a temperature above 190°C and below 200°C, through live steam injection, because, above this temperature, the glycerol can polymerize and also decompose itself. The separation of glycerol from water is made by condensation with controlled temperature, providing the separation of these two miscible components.

The obtained glycerol, in this condensation process, present up to 99% of purity to eliminate de odors, the glycerin pass through a process of deodorization in a high vacuum vessel (flash) with drag steam, following to the process of clarification com activated coal and filtration to its elimination. In this process, it is also obtained an impure glycerol of lower commercial value, which is employed with the denomination of technical or industrial glycerin.

The purification by ion exchange aims the extraction of existent impurities in the glycerin, such technique consisting of passing by successive catalytic beds made of strong cationic resin and weak anionic resins. Just after that, the entire product is passed by successive evaporators.

Approximately 300,000 tons per year of blond glycerin are generated in the production of biodiesel due to B5 that is mandatory the mixture of 5% of biodiesel in mineral diesel. An expectative from 2013 of entry of B7 that would elevate this volume to about de 420,000 tons per year of blond glycerin exists. The blond glycerin contains inorganic salts, water, residual of monoglycerides and it is the raw material used to the distillation, many times bidistillation, with post-treatment with absorbents systems, usually activated coal to produce the distilled glycerin, which now is consumed in the market. Basically, all the glycerin used by food, pharmaceutical, paint and varnish industry. The value of the market of blond glycerin is very low because the production of distilled glycerin is expensive, typically the value of the distilled glycerin is 3 to 4 greater than the blond glycerin, it has a high-energy demand, the typical process of distillation uses temperatures from 220°C to 250°C under negative pressure. It is necessary investment in a specific plant for production of distilled glycerin. The national demand of distilled glycerin is of approximately 50.000 tons per year, all the remaining glycerin is exported with blond glycerin to China with very low values. In the first semester of 2013, about 90,000 tons of blond glycerin were exported.

With this current scenario of market and existent technologies that really can be implemented industrially, an innovative process was developed to produce glycerin with minimum purity of 96%, that does not need distillation, from crude glycerin generated in the processes of transesterification of the biodiesel plants. Such process has a very low energy cost compared to the distillation, it generates less residue, it does not use any chemical input, as chemical solvent, and that can be integrated in the existent biodiesel plants using alkaline catalysis, without the need of investments in a distillation plant. Such process use as mechanism of purification the liquid/liquid extraction, where it is vital the knowledge of the components and adequate its relations to obtain the maximum yield and the maximum purity to the produced glycerin.

### Prior art

Since the current invention aims the method of glycerin purification from biodiesel, great efforts have been done to improve the techniques of purification and reduce the energy costs and generation of effluents.

The invention WO2011/033346 aims to produce biodiesel from waste oils (rest of household and restaurant oils) using alcohols such as ethanol and methanol in the presence of an acid resin reticulated to esterification and to bring acidity to a level from 1 to 2 mgKOH/g and then make a transesterification with homogeneous basic catalyst of metallic hydroxides, alkoxides and oxides, Biodiesel and glycerin.

Since the type of fatty acid and catalyst interferes directly in the production of both fuel and glycerin, it is interesting to lower the cost not affecting the yield, and in the case of enzymes, try to do its reuse. The document US 8 012 713 refers to a method of using the crude glycerin as a carbon source to the development of proteins and enzymes that can be used in many ways, since their direct consumption up to their production in large scale to be used as enzymes.

A brief search in the database on documents that support the understanding of the present invention is listed below. The Brazilian document n° PI 0603857 (UNICAMP) comprises the production of alkyl esters, such as Biodiesel, from any vegetable oil or animal fat, in methyl or ethyl routes, catalyzed by modified strong base, both in homogeneous phase, which provides high yield, in the reaction of transesterification, promoting the spontaneous decantation of glycerin. After removal of excess alcohol from reaction medium, the neutralization of catalyst and its purification, esters are obtained (biodiesel) with purity above 99%. The catalysts of the present invention allow both batch and continuous process, where it can be used all the engineering of industrial plants that already operate with Methanol to operate in the ethyl route. The heterogeneous phase catalysts simplify the process, because they do not contaminate the products, not requiring the steps of neutralization and purification of alkyl esters and they are not consumed during the process of transesterification.

Another document n° PI 0703303-6 (INT: *Instituto Nacional de Tecnologia* National Institute of Technology) refers to a process for obtaining Biofuels from liquid or solid triacylglycerides at room temperature with different average molecular weights and maturation degrees, from vegetable or animal origin, crude or refined, mono alcohols of different molecular weights, and homogeneous or heterogeneous catalysts. Such invention is characterized by the introduction in the reaction medium of Diesel oil, kerosene and/or its mixtures available in the national and international markets. In addition, the invention provides the reduction of viscosity of reaction medium, with elevation of the velocity and of yield of conversion, facilitates the separation of glycerin and residuals of alcohol and catalyst employed, and promotes, in a general way, the obtaining of products more in accordance with the respective specifications to Biodiesel B100.

Another Brazilian document n° PI 0902550-2 (SENAI) discloses a practical method of blond glycerin purification, from the production of biodiesel, free of distillation to reduced pressure and without need of neutralization of the medium via chemical agents ranked as organic or inorganic bases. In this process of purification, acetals and cetals are produced from cyclization of the respective triol in the presence of different aldehydes and ketones in the absence of additional acid catalysts, the cyclized adducts being hydrolyzed in different concentrations of distilled water and the glycerin recovered with degree of purity that varies from 90 to 99.9%, in addition to the chemical yield is quantitative. Although this technique is distinct of direct distillation, it uses a chemical input (aldehydes or ketones) to prepare an intermediate that is the acetal, which need to be separated by some technique (distillation or crystallization) and later this need to be hydrolyzed with water to generate glycerin, aldehydes or ketone, and water. The cost of this process can be greater than of the direct distillation of glycerin with salts, in addition to using flammable solvents.

### Summary of the invention

In order to eliminate the drawbacks reported above, more specifically provide a direct production of glycerin 96- 99.5% free of inorganic salts, using the crude glycerin originated in the processes of transesterification that use alkaline catalysis, these are the basic steps that allowed the development of this patent:
1- analyzing all components of crude glycerin, mainly the content of free glycerin, combined glycerin, fatty soaps, esters, mono- and diglycerides, free alkalinity, free methanol and humidity. These 2 latest components can be removed before or after the process of purification via distillation.
2- unbalancing the composition of homogeneous crude glycerin using the own fatty material recovered from crude glycerin or using fatty acid coming from acidification of alkaline sludge, fatty acids from the process of physical refining of triglycerides, or even distilled fatty acids.
3- separating by physical decantation in continuous process or by batch processes in 2 different higher phases composed essentially of fatty materials (free fatty acids, fatty soaps, esters, mono/diglycerides), lower phase, which is the heavy phase composed essentially of free glycerin and residual of fatty soaps and partial glycerides.
4- determining the content of fatty soaps of higher phase and using inorganic acids (hydrochloric acid, sulfuric acid, phosphoric acid) or short carboxylic acids (citric, oxalic, lactic, formic, acetic) make the chemical breakdown of fatty soaps converting them into their respective fatty acids. Adding water to wash the salts formed and separating via physical decantation, where the higher phase is aqueous solution and the lower phase is the phase containing materials free of soaps. Part of this phase of fatty material is recycled back to the process of purification to create the unbalance of crude glycerin, which is in homogeneous phase.

The present development makes the process innovative, extremely competitive and sustainable from the economic and environmental point of view:
a- Main phase of the process: transforming the crude glycerin, which is in homogeneous phase into two different phases. The objective is to make the unbalance of the homogeneous mixture of fatty material with the main component free fatty acid that can be the own acid generated by this process. The crude glycerin, which is a homogeneous mixture, with the addition of unbalance agent, is separated into two phases, higher phase that contains the fatty materials contained previously in the crude glycerin and lower phase that contains the glycerin of 96% to 99.5% purity;
b- the process does not use any additional chemical compound to the already existing in the producing plants of biodiesel
c- the process recovers and does not generate any type of effluent, which transform it in the optimal process to the plants of biodiesel
d- the energy consumption to the production of glycerin 96% is much lower compared to the traditional process of distillation
e- the glycerin obtained by this process avoids the risk of having toxic microcomponents (for example acrolein) as can occur during the distillation of glycerin. Additionally to this fact, the glycerin obtained by this process does not destroy the natural components of tocopherol type existent mainly in the glycerin obtained from production plants of biodiesel that use soybean and sunflower oils.
f- the process ensures the utilization of 100% of all fatty material used by producer of biodiesel, which from the economic point of view is extremely important since biodiesel is a commodity and losses, no matter how small they are, can derail its operation from de economic point of view.
g- the process creates 2 new types of glycerin to the consuming market, *GLICERINA PUR* [GLYCERIN PUR] 96 and *GLICERINA PUR* [GLYCERIN PUR] 99.

The type of glycerin so-called *GLICERINA PUR* 96 can be used in the industrial applications:
a- Production of alchemical resins to paint industries
b- production of monoacetins, diacetins and triacetin used as humectants and solvents in the adhesive, ceramic, tobacco industry
c- production de acetals, solvent no petrochemical used in the industry in general
d- production of surfactant monoglycerides and diglycerides used in the food, paint and varnish industry, polymers
e- used as humectants in the agricultural industry and tobacco industry
f- production of propileneglycol, acrylic acid, acrilonitrile
g- production of polyglycerins, input to the production of surfactants
h- as component of cooling fluids used by automotive industry
i- production of plasticizers to PCV and rubber industry
j- in the production of epoxy resins used in the adhesive industry, heat isolation, paints and varnishes.

*GLICERINA PUR* 99 can be used:
h- In the applications mentioned of *GLICERINA PUR* 96 (items a up to i); however, its degree of purity is higher and its cost of purification is higher due to the needed additional step.
i- As components in cosmetic, pharmaceutic and food formulations
j- In the production of soaps based on glycerin
k- Production of toothpastes

### Representatives examples of the art

### Example 1-GLICERINA PUR 96;

Step 1: Crude glycerin originated from a commercial plant of biodiesel that use soybean oil as raw material was analyzed having the following composition: 26.4% free glycerin, 36.0% water, 35.0% free methanol, fatty soaps 4.2%, 1.3% partial esters and glycerides, free alkalinity 9.0 mgkoh/grams, 3500 grams of this product was added 160 grams of fatty acid recovered with acidity index 180mgkoh/grams and the free methanol and water was removed by distillation at 110°C under reduced pressure of 50 mmhg. After this step, a mass of 1175 grams of dry product with the following composition was obtained: 76.4% total glycerin, 1.0% water, free methanol, 21.5% fatty soaps, 1.1 % partial esters and glycerides, free alkalinity.

Step 2: in the reaction mass of step 1 780 grams of fatty acid recovered with acidity index 180mgkoh/grams were added. The mixture was done at 70°C, agitated for 15 minutes, after this time, the agitation was stopped and kept during 60 minutes to obtain the complete separation of higher phase and lower phase. The lower phase was separated physically by decantation, about 877 grams of purified glycerin was obtained with the following composition: *96.5% total glycerol,* fatty soaps, 1.9% partial esters and glycerides, 1.6% water, color Gardner 10.5. The glycerin extraction yield of the process was 93%.

Step 3: The remaining higher phase in the mixer of 1050 grams with the following composition: total glycerol of 5.98%, 38% fatty soaps, fatty acids, esters, 56% partial glycerides%; 55 grams of water agitated for 15 minutes were added and maintained in decantation for 60 minutes. 102 grams of glycerin water containing 48% of total glycerol, which later is recycled in crude glycerin before removal of methanol and water. With this additional recovery, the yield of total glycerol rises to 98.2%. The material phase containing 1.5% of total glycerol was treated with a solution 433 grams of 30% phosphoric acid solution, agitated during 30 minutes at 70°C and lower phase was decanted and separated, obtained about 500 grams of a transparent liquid solution almost colorless containing 38% of sodium monophosphate that later can be sent as component to acidified phase A fertilizers of 984 grams composed basically of free fatty acids with acidity index 183 mgkoh/grams is recycled as extraction agent of initial process.

### Example 2 - GLICERINA PUR 96:

Step 1: Crude glycerin originated from a commercial plant of biodiesel that use soybean oil as raw material was analyzed having the following composition: 26.4% free glycerin, 36.0% water, 35.0% free methanol, 4.2% fatty soaps, 1.3% partial esters and glycerides, free alkalinity 9.0 mgkoh/grams, 1750 grams of this product were added 80 grams of recovered fatty acid with acidity index 180mgkoh/grams and the free methanol and water were removed by distillation at 110°C under reduced pressure of 50 mmhg. After this step was obtained, a mass of 588 grams of dry product with the following composition: 76.4% total glycerin, 1.0% water, free methanol, 20.5% fatty soaps, 1.1 % partial esters and glycerides, free alkalinity.

Step 2: in the reaction mass of step 1, 390 grams of recovered fatty acid with acidity index 180mgkoh/grams were added. The mixture was done at 70°C, agitated for 15 minutes, after this time the agitation was stopped and maintained during 60 minutes to obtain the complete separation of higher phase and lower phase. The lower phase was separated physically by decantation, about 439 grams of purified glycerin were obtained with the following composition: *96.5% total glycerol,* fatty soaps, 1.9% partial esters and glycerides, 1.6% water, color Gardner 10.5. The glycerin extraction yield of the process was 93.5%.

Step 3: The remaining higher phase in the mixer of 525 grams with the following composition: total glycerol of 5.98%, 38% fatty soaps, fatty acids, esters, 56% partial glycerides%; were added 28 grams of water agitated for 15 minutes and kept in decantation for 60 minutes. 50 grams of glycerin water were separated containing 51% of total glycerol that later is recycled in crude glycerin before removal of methanol and water. With this additional recovery, the total glycerol yield rises to 98.6%. The material phase containing 1.4% of total glycerol was treated with a solution of 30 grams of 98% sulfuric acid, 30 grams of water agitated during 30 minutes at 70°C and lower phase was decanted and separated, about 80 grams of wash water was obtained and sent to appropriate treatment. The acidified phase of 490 grams composed basically of free fatty acids with acidity index 185 mgkoh/grams is recycled partially as extraction agent of initial process.

### Example 3 - GLICERINA PUR 96:

Step 1: Crude glycerin originated from a commercial plant of biodiesel that uses soybean oil as raw material after distilling the free methanol and water was analyzed having the following composition: 72.8% total glycerin, 0.32% water, free methanol, 25.6% fatty soaps, 1.27% partial esters and glycerides. About 1460 grams of this product were added 1468 grams of recovered fatty acid with Acidity index 188mgkoh/grams

Step 2: in the reaction mass of step 1 were added 1468 grams of recovered fatty acid with acidity index 188mgkoh/grams. The mixture was done at 70°C, agitated for 15 minutes, after this time, the agitation was stopped and kept during 60 minutes to obtain the complete separation of higher phase and lower phase. The lower phase was separated physically by decantation, about 1040 grams of purified glycerin were obtained with the following composition: 96.8% *of total glycerol,* fatty soaps, 2.2% partial esters and glycerides, 1.0% water, color Gardner 7.0. The glycerin extraction yield of the process was 94.6%

Step 3: The remaining higher phase in the mixer of 1890 grams with the following composition: glycerol total of 1.02%, fatty soaps of 18.8%, fatty acids, esters, partial glycerides of 80.0% was acidified with 140 grams of 30% hydrochloric acid and kept in agitation during 30 minutes at 70°C, agitation was stopped and after 60 minutes the lower phase was decanted and separated as wash water. The acidified phase of 1760 grams composed basically of free fatty acids with acidity index 189 mgkoh/grams is recycled partially as extraction agent of initial process.

### Example 4 - GLICERINA PUR 96:

Step 1: Crude glycerin originated from a commercial plant of biodiesel that uses fridge sebum as raw material after distilling the free methanol and water was analyzed having the following composition: 58% total glycerin, 1.55% water, free methanol, 23.0% fatty soaps, 19.0% partial esters and glycerides. About 319 grams of this product were added in the mixer.

Step 2: in the reaction of step 1 were added 480.0 grams of recovered fatty acid with Acidity index 160mgkoh/grams. The mixture was done at 80°C, agitated for 15 minutes, after this time, the agitation was stopped and kept during 60 minutes to obtain the complete separation of higher phase and lower phase The lower phase was separated physically by decantation, about 170 grams de purified glycerin were obtained with the following composition: 93.0% total glycerol, fatty soaps, 3.5% partial esters and glycerides, 3.5% water, color Gardner 9.0. After additional drying, the content of *total glycerol rises to* 96.5%. The glycerin extraction yield of the process was 86.0% in this step

Step 3: A remaining higher phase in the mixer of 616 grams with the following composition: total glycerol of 4.4%, 9.4% fatty soaps, fatty acids, esters, partial glycerides of 86,2% was acidified with 23 grams of 30% hydrochloric acid and kept in agitation during 30 minutes at 60°C, agitation was stopped and after 60 minutes, the lower phase was decanted and separated as wash water. The acidified phase of 560 grams composed of a mixture of fatty acids, esters, partial glycerides with acidity index 135 mgkoh/grams is recycled partially as extraction agent of initial process.

### Example 5 - GLICERINA PUR 99:

Purified glycerin of example 1 with composition of 96.5% *total glycerol,* fatty soaps, 1.9% partial esters and glycerides, 1.6% water, color Gardner 10.5 was continuously passed by lab column of double cover containing 80% of powder-activated coal, with 10% of montmorillonite type clay, 10% of silica brand Trisyl, previously mixed. The column placed horizontally and the glycerin were kept at 90°C using a thermostat circulation bath and gravitationally were passed in the column. The treated glycerin by this method is odorless, colorless, *total glycerol of 99.1%,* and fatty material residual of 0.7%, humidity of 0.2%, totally free of mineral salts.

With a crude glycerin and the fatty acid recovered of example 4, about 20 batches were performed to simulate a continuous operation always recycling the recovered fatty acid of step 3 and feeding back in step 2. The content of total glycerol total of purified glycerin free of humidity was from 93.0% to 98% and the recovery yield was from 86% to 97%. The same procedure was done with the dry glycerin originated from soybean oil and recovered fatty acid of example 1. The content of total glycerol of purified glycerin free of humidity was from 96.5% to 98% and the recovery yield was from 94% to 98%.

With these 40 experimental data and more 40 additional experimental data using several crude glycerin of producing plants of biodiesel in Brazil, a predictive model of glycerin purity and final recovery yield of this process was built, which is the database called *GLICERINA PUR DATA PREDICT,* essential to determine the optimum relations between the components of crude glycerin and recovered or pure fatty acid to maximize yield and purity of this process.

## Claims

1. Process of crude glycerin purification originated from transesterifications using alkaline catalysis without using acidification and distillation producing purified glycerin 96% and 99% purity, **characterized in that** the process is applied to crude glycerin from industrial plants using alkaline catalysis in the hydroxide, oxides and alkoxides form, more specially those based in sodium, potassium and lithium methylate producing, as final product, the following types of glycerin.
- purified glycerin without using physical distillation with minimum purity of 96%; and
- purified glycerin without using physical distillation with minimum purity of 99%.

2. Process according to claim 1, wherein the steps are:
a- analyzing all components of crude glycerin, mainly the content of free glycerin, combined glycerin fatty soaps, esters, mono- and diglycerides, free alkalinity, free methanol and humidity; wherein the free methanol and humidity can be removed before or after the process of purification via distillation;
b- unbalancing the composition of homogeneous crude glycerin using the own fatty material recovered from crude glycerin or using fatty acid coming from acidification of alkaline sludge, fatty acids coming from the process of triglycerides chemical refining, or even distilled fatty acids;
c- separating, by physical decantation in a continuous process or by batch processes, in 2 different phases: higher phase composed essentially of fatty materials (free fatty acids, fatty soaps, esters, mono/diglycerides), lower phase or heavy phase composed essentially of free glycerin and residual of fatty soaps and partial glycerides;
d- determining the content of fatty soaps of the higher phase and using inorganic acids (hydrochloric acid, sulfuric acid, phosphoric acid) or short carboxylic acids (citric, oxalic, lactic, formic, acetic);
e- making the chemical breakdown of fatty soaps converting into their respective fatty acids; and
f- adding water to wash the salts formed and separating via physical decantation, wherein the higher phase is aqueous solution of salts and the lower phase is the phase containing materials free of soaps, part of this fatty material phase is recycled back to the process of purification to create the unbalance of crude glycerin, which is in homogeneous phase.

3. Process according to the previous claims, wherein the temperature of extraction during the separation is from 40°C to 95°C, preferably between 60°C and 80°C.

4. Process according to the previous claims, wherein the mixture of fatty materials containing soaps is acidified with mineral acids or short carboxylic acid to release the free fatty acids, preferably using phosphoric acid to generate a solution with sodium monophosphate.

5. Products of purified glycerin 96% and 99% purity, wherein the crude glycerin from the process according to claims 1 and 2 without the process of acidification using inorganic acids such as sulfuric acid, hydrochloric acid, phosphoric acid and/or short carboxylic acids such as lactic, oxalic, and citric acid, comprises a composition of contents of total glycerin from 20% to 90%, fatty materials in the form of soaps, esters, partial and volatile glycerides from 2% to 45%, compound of water and free alcohol from 0% to 70%, free alkalinity from 0 to 80 expressed in milligram of potassium hydroxide per 1 gram of product.

6. Products of purified glycerin 96% and 99% purity according to the previous claims, wherein the extraction agent or the initially homogeneous system unbalance existent in the crude glycerin are fatty acids in the pure form or their mixtures thereof with esters and/or partial glycerides with content from 30% to 100% of carboxylic fatty acids.

7. Products of purified glycerin 96% and 99% purity according to claim 6 and in an preferential option, wherein it uses the fatty acid recovered from own process, the composition of which is between 60% to 100% of compounds of carbon chain C12 to C20, preferably composed of saturated or unsaturated fatty acids of carbon chain C16 and C18.

8. Products of purified glycerin 96% and 99% purity according to the previous claims, wherein the mass relation between the extraction agent - crude glycerin of dry base used-is from 3.0 to 0.2, preferably from 1.5 to 0.50.

9. Products of purified glycerin 96% and 99% purity according to the previous claims, wherein the yield of glycerin extraction relative to its initial content is from 85% to 98% by mass, more specifically above 90% depending on the quality of crude glycerin used, consequence of different processes used by biodiesel producers or industrial fatty esters.

10. Products of purified glycerin 96% and 99% purity according to previous claims, wherein the glycerin with purity above 99% is obtained with an additional treatment using filter clays and active coal.
